# EUROPEAN PATENT SPECIFICATION

(11) **EP 0 192 401 B1**
(45) Date of publication and mention of the grant of the patent: **13.01.1993**
(21) Application number: 86300932.0
(22) Date of filing: 11.02.1986
(51) Int. Cl.: C12N 9/40

(54) **Polypeptide product**
Polypeptid-Produkt
Produit polypeptidique

(30) Priority: 12.02.1985 DK 629/85
(43) Date of publication of application: 27.08.1986
(73) Proprietor: NOVO NORDISK A/S, 2880 Bagsvaerd (DK)
(72) Inventor: Nielsen, Ruby Ione, Dk-3520 Farum (DK); Schülein, Martin, DK-2100 Copenhagen (DK)
(74) Representative: Brown, John David

(56) References cited:
- EP-A- 0 121 960
- FR-A- 2 259 900
- US-A- 4 332 894
- BIOLOGICAL ABSTRACTS, vol. 71, no. 5, 1981, Philadelphia, PA (US); I.V.ULEZLO et al., p. 3214, no. 30707

## Description

The present invention relates to novel, microbially derived α-galactosidase products. The novel enzyme has been named α-galactomannangalactosidase.

### BACKGROUND OF THE INVENTION

D-Galacto-D-mannans are reserve carbohydrates found in the endosperms of some legume seeds. Galactomannans consist of a β-D-(1-4)-mannan polymer backbone to which various amounts of single (1-6)-α-D-galactopyranosyl groups are attached.

Galactomannans are employed as ingredients of food-stuffs and of animal feed and for cosmetic, pharmaceutical and other industrial applications. Typically, galactomannans are used for the production of interactive gels by addition of polymers such as xanthan gum, carrageenan gum, agar and agarose to the galactomannan. This interaction is hereinafter referred to as gelling capacity. However, if the ratio between galactose and mannan in a galactomannan is too high, gels are poorly or not formed, this hereinafter being referred to as poor or no gelling capacity.

The problem of poor or no gelling capacity may be solved by removing some of the galactopyranosyl groups from the galactomannans using, for example, α-galactosidases recovered from plants, such as lucerne seed α-galactosidase, vide Carbohydr.Res. 71 (1979), 212, and 92 (1981), 269. Also, other α-galactosidases recovered from plants have comparable activity. See, for example, U.S. Patent No. 4,332,894. However, α-galactosidases recovered from plants are relatively expensive.

In order to prepare galactomannans having a sufficiently low content of galactopyranosyl groups to permit satisfactory interactive gel formation, it is necessary to release at least about 10% (weight/weight) of the galactose content from galactomannans, such as guar galactomannan. Percentages of galactose liberated from a galactomannan given herein are based upon the content of galactose in the galactomannan starting material unless otherwise indicated.

In order to prepare galactomannans having a desired low ratio between galactose and mannan, the enzymatic removal of the galactopyranosyl groups may be performed in a reaction medium having a high content of galactomannans. Preferably, the content of galactomannans in the reaction medium is in the range from 2 to 70% (weight/weight), preferably from 8 to 50% (weight/weight), cf. European patent application No. 84200304.8. However, the enzymatic removal of the galactopyranosyl groups can also conveniently be performed in a medium containing less than 2% galactomannan.

It has been reported (see J.Bacteriol. 129 (1977), 850 et seq., and J.Biol.Chem. 224 (1969), 2975 et seq.) that α-galactosidase from Aspergillus niger hydrolyzed guar flour and locust bean gum. This result could be due to the Aspergillus niger enzyme used being contaminatad with a substantial amount of endo-β-mannanase, which first splits the mannan backbone of the galactomannan and thereby permits attack by the α-galactosidase. Galactomannans prepared from the known Aspergillus niger enzyme will have poor or no gelling capacity. It is stated at the bottom of page 7 in European Patent Application No. 84200304.8 that Aspergillus niger-α-galactosidase hydrolyzed guar galactomannan which had been depolymerized with β-mannanase. β-Mannanase is an enzyme which is produced by most fungi. β-Mannanase degrades β-D-(1-4)-mannan linkages.

It is stated in J.Biol.Chem. 245 (1970), 786, that Mortierella vinacea-α-galactosidase does not hydrolyze D-galactose from the galactomannan of leguminous seeds. Also, α-galactosidase I and α-galactosidase II from Bacteriodes ovatus were unable to remove galactose residues from intact guar gum, see J.Bacteriol. 161 (1985), 500.

Several other microbial α-galactosidases are known to the art. The inventors hereof have screened for α-galactosidase production about 200 different strains of fungi and bacteria selected from most of the saprophytic genera including those listed in Appl.Biochem.Microbiol. 18 (1982), 1 et seq. All those tested were found to be unable to split off D-galactopyranosyl groups from galactomannans, if the mannan backbone was not degraded.

### OBJECTIVES OF THE INVENTION

One object of this invention is to provide microbial α-galactosidases able to split off a significant amount of galactose from galactomannans without substantially degrading the mannan backbone. The novel α-galactosidases are hereinafter designated α-galactomannangalactosidases. Their action improves the gelling capacity of galactomannans.

A second object of this invention is to provide α-galactomannangalactosidase products containing little or no endo-β-mannanase. Substantial degradation of the mannan backbone destroys the gelling capacity.

A further object of this invention is to provide microbial α-galactomannangalactosidases which in a reaction medium containing a relatively high concentration of the galactomannans are able to prepare galactomannans having improved gelling capacity. Such microbial source α-galactomannangalactosidases are desired for several reasons. First, they are not so expensive as α-galactosidases recovered from plants. Second, use of a reaction medium containing a relatively high content of galactomannans is also important due to economical considerations.

### BRIEF STATEMENT OF THIS INVENTION

It has now surprisingly been found that certain Penicillium species elaborate α-galactomannangalactosidase enzymes. The invention relates to this novel enzyme, the method for its production and the method of its use.

### DETAILED DISCUSSION OF THIS INVENTION

The α-galactomannangalactosidase products of this invention are not the same as the α-galactosidases heretofore known. The difference between the known α-galactosidases and the α-galactomannangalactosidase of this invention can, inter alia, be shown by immunochemical tests and through property differences.

The α-galactomannangalactosidase products of this invention are able to cleave off 1,6-α-D-linked galactopyranosyl residues from galactomannans such as guar gum in a reaction medium having a relatively high content of the galactomannans without substantially degrading the mannan backbone. Preferably, the α-galactomannangalactosidase products of this invention are essentially free of endo-β-mannanase activity measuring less than 0.01 EMANU per GALU, preferably less than 0.001 EMANU per GALU, according to the Activity Tests being described hereinafter. These limits are emperical because they involve extensive purification procedures in order to remove endo-β-mannanase from the desired product; the limits represent the degree to reduction of endo-β-mannanase content necessary to permit the use of the α-galactomannangalactosidase for preparing galactomannans having desired gelling capacities. The α-galactomannangalactosidase products purified as described in Example 9 below were suited for preparing galactomannans having a desired gelling capacity.

The enzymes characteristics are as follows: The molecular weight is between 55,000 and 67,000. The pH optimum is in the range of 4 to 6. The pI value is about 4.3. The temperature optimum is 55°C (short term) and 50°C (long term), see Example 10 for further details.

Using the α-galactomannangalactosidase products of this invention, galactomannans with the ability to form gels of various strengths ranging from very weak gels to strong gels are formed by appropriate selection of the extent to which the galactopyranosyl groups are split off. The extent to which the galactopyranosyl groups are split off may be controlled by adjusting the length of the time that the galactomannans are treated with the α-galactomannangalactosidase products of this invention and by appropriately selecting the concentration of the α-galactomannangalactosidase products of this invention. The α-galactomannangalactosidase products of this invention are able to split off at least 10% (weight/weight) based on the galactomannan. Some preferred α-galactomannangalactosidase products of this invention are able to split off 20% (weight/weight) and even 50% (weight/weight) of the galactose from the galactomannan without substantially degrading the mannan backbone.

Thus, the incubation may, for example, be stopped when the galactose content in the resulting galactomannans reaches the range from 1 to 50% (weight/weight), especially from 10 to 25% (weight/weight), of the original galactose content. Because the α-galactomannangalacotsidase products of this invention are essentially free from endo-β-mannanase activity, it is possible to split off galactopyranosyl residues from galactomannans without substantially degrading the mannan backbone which means that it is possible to prepare galactomannans having, on one hand, an average molecular weight of at least 50,000, preferably at least 500,000, most preferred at least 1,000,000, and, on the other hand, a desired gelling capacity. To repeat, galactomannans having low molecular weight exhibit very low or no gelling capacity.

The treatment of galactomannans with the α-galactomannangalactosidase products of this invention is preferably performed in a reaction medium containing at least 1% (weight/weight), preferably at least 2% (weight/weight), most preferred at least 30% (weight/weight), of the galactomannans. The use of a reaction medium containing large amounts of galactomannans is preferred for economical considerations. The reaction medium is, preferably, aqueous.

Examples of the most important galactomannans are quar galactomannan (obtainable from Cyanaposis tetragonolibus) and locust bean gum (obtainable from Ceratonia siliqua). Further examples are stated in Adv.Carbohydr.Chem.Biochem. 35 (1978), 341 et seq. Common galactomannans contain between about 20 and 50% (weight/weight) galactose, often between about 35 and 45% (weight/weight) galactose. Guar galactomannan contains about 35% of galactose and about 62% of D-mannose and it is believed to have a molecular weight between about 16 x 10⁵ and 22 x 10⁵. Locust bean gum contains between about 20 and 25% (weight/weight) of galactose and between about 75 and 80% (weight/weight) of mannose and is believed to have a molecular weight between about 11 x 10⁵ and 16 x 10⁵.

Specific examples of products gelated with galactomannans are food and feed products, for example, soft drinks, bakery jelly and instant chocolate pudding, vide Food Technol. 27 (1973), 26 et seq., and pet food.

The α-galactomannangalactosidase products of this invention can be prepared by surface cultivation or by submerged cultivation of a microorganism, for example, α-galactomannangalactosidase producing fungi or bacteria, preferably fungi, followed by recovery of the enzyme by conventional techniques.

The microorganism to be used can be chosen by any skilled art worker using the following four criteria for the selection:
1) The enzyme prepared by the microorganism must have α-galactosidase activity; see the Activity Test for p-nitrophenyl-α-D-galactopyranoside, below;
2) The enzyme prepared by the microorganism should have an initiation rate of at least 0.3 g galactose/(litre x hour x GALU) using the screening method with guar G described in Example 8 below.
3) The enzyme product prepared by the microorganism must have an endo-β-mannanase activity below 0.01 EMANU per GALU, see the Activity Tests below.
4) The enzyme prepared by the microorganism must be able to improve the gelling capacity of guar gum so that a gel can be formed using the test method described in Example 10 below.

Surface cultivation is carried out on a semi-solid medium containing an assimilable nitrogen source and carbon source and essential nutrients.

Submerged cultivation is carried out under aerobic conditions in a fermentation medium comprising an assimilable nitrogen source and carbon source and essential nutrients. Further details such as pH value, temperature, aeration and agitation, can easily be selected by the skilled art worker.

The α-galactomannangalactosidase herein exemplified is an extra cellular enzyme elaborated by strains of well-known species, namely Penicillium chrysogenum, Penicillium islandicum and Penicillium notatum. Exemplary strains are, respectively, DSM 3214, DSM 3215 and DSM 3216.

The recovered α-galactomannangalactosidase can be used as such or can be further purified, for example, by ion exchange chromatography, fractional precipitation or gel filtration, to remove, inter alia, endo-β-mannanase. Preferably, the α-galactomannangalactosidase products of this invention will exceed about 250 GALU/ml in activity of enzyme solutions (such being far more concentrated as has ever been found in cultures) or 250 GALU/g if in solid form.

The α-galactomannangalactosidase can also be obtained from strains which have been mutated so that they produce a minor amount or no endo-β-mannanase. Methods of mutating strains are known by the skilled art worker, for example, ultra violet radiation and chemical mutagens.

This invention is further illustrated by the following examples which, however, are not to be construed as limiting. The examples illustrate some preferred embodiments of the invention. The letters "TM" indicate that it is a trade mark.

In order to prepare α-galactomannangalactosidase products according to this invention, the products obtained according to Examples 1 - 6 below were further purified as described in Example 9.

The strains used in Examples 1 - 3 were deposited at Deutsche Sammlung von Mikroorganismen (DSM) in January 1985.

### Cultivation of Strains

The α-galactomannangalactosidase producing strains may be grown on agar slants containing the following ingredients per litre: 4.0 g of yeast extract, 1.0 g of potassium dihydrogen phosphate, 0.1 g of magnesium sulphate, heptahydrate, 15 g of glucose and 20 g of Bacto^{TM} (Difco Laboratories, Detroit, U.S.A.) agar. This substrate is autoclaved at 121°C for 20 or 40 minutes and will, hereinafter, be referred to as YPG.

### Activity Test for α-galactosidase

The principle of the test is that α-galactosidase hydrolyses the colourless p-nitrophenyl-α-D-galactopyranoside (hereinafter designated p-NPGal) forming p-nitrophenol which is yellow when alkaline. One α-galactosidase unit (hereinafter designated GALU) is the amount of enzyme which hydrolyses 1 µmol p-NPGal per minute under the following standard conditions: Substrate: 0.80 mM of p-NPGal, pH: 5.5 (0.0333 M acetate buffer), temperature: 37°C and reaction time: 15 min. The colour formed is measured by spectrophotometri at the wavelength 405 nm.

Further details of this method are described in the leaflet AF 204/2 GB dated 29 November 1984 from Novo Industri A/S.

### Activity Test for endo-β-mannanase

The endo-β-mannanase activity was determined by the reducing effect on the viscosity of locust bean gum using Gamanase^{TM} 1.5 L (Novo Industri A/S) as standard. The method is described in the leaflet AF 156/1 GB dated 16 September 1975 from Novo Industri A/S. The activity unit referred to in this leaflet is called HCU. The activity of Gamanase 1.5 L is 1,500,000 HCU/g. By assaying the amount of reducing sugar released, using the Somogyis Nelson Method (J.Biol.Chem. 153 (1944), 375) and using mannose as a standard, it has been found that 1 HCU corresponds to 0.011 EMANU where 1 EMANU is defined as the amount of endo-β-mannanase which releases 1 micromole mannose equivalent from locust bean gum per minute at 50°C and at a pH value of 5.0.

### Example 1

### a) Enzyme Production

A substrate for shake flasks was prepared with the following ingredients per litre: 20 g of cornsteep liquor (Corn Products Corp., 10 g of ammonium nitrate, 10 g of potassium dihydrogen phosphate, 1 g of magnesium sulphate, heptahydrate, 10 g of guar gum extra LK (NDH Nordisk Droge Handels A/S, Denmark), 0.1 g of Pluronic^{TM} L61 (BASF, Federal Republic of Germany), 5 g of calcium carbonate and tap water. The pH was adjusted to 6.5 and sterilization took place at 121°C for 40 minutes. A 500 ml Erlenmeyer flask with 100 ml of substrate was inoculated with about 10⁷ spores from a YPG agar slant previously inoculated with Penicillium chrysogenum DSM 3214. The flasks were shaken at 230 rpm and at 30°C for 3 or 4 days whereafter the fermentation broth was centrifuged. The supernatant containing the α-galactomannangalactosidase was separated from the mycelium. The mycelium was discarded and the supernatant was analysed for α-galactosidase. Under these conditions the strain produced 10 GALU/ml.

### b) Enzyme Purification

1 litre of culture broth was filtered through a glass fibre filtre (Whatman^{TM} GF/D and GF/F) on a Büchner funnel with a diameter of 11 cm. The filtrate was concentrated to 350 ml by osmosis with an Amicon^{TM} Hollow fibre cartridge (HlP10) having a cut off at a molecular weight of 10,000, whereafter, the concentrate was washed out with deionized water until the conductance was 3 mSi (milliSiemens). Thereafter, the concentrate was concentrated to 50 ml using an Amicon^{TM} cell with a GR81PP membrane (De Danske Sukkerfabrikker, Denmark) and having a cut off at a molecular weight of 6,000. In the concentrate obtained, pH was adjusted to 7.0.

A DEAE-Tris acryl ion exchange column was equilibrated with 50 mM Tris buffer (pH: 7.0). (Tris is tris(hydroxymethyl)aminomethane.) 200 ml of the ion exchanger was packed in a column having a diameter of 5 cm and the column was packed so that it had a flow rate of 320 ml per hour. Ion exchange was performed on the filtrate from the glass filter and fractions were collected. The α-galactomannangalactosidase binds weakly to this ion exchanger and was eluted either after washing with Tris buffer or with a sodium chloride gradient. The activity of α-galactomannangalactosidase in the fractions was measured and the fractions containing protein with this activity were collected and concentrated.

The proteins were concentrated and low-molecular weight components were washed out on an Amicon^{TM} Hollow fibre cartridge (HlP10) with a cut off by molecular weight 10,000.

The resulting 10 ml enzyme solution was analyzed by measuring the activity of α-galactomannangalactosidase and of endo-β-mannanase. The activities found were 300 GALU/ml and 23 EMANU/ml (2100 HCU/ml), respectively.

### Example 2

Using the same procedure as described in Example 1a, with the proviso that the strain used was Penicillium islandicum DSM 3215, the yield was 17 GALU/ml.

Purifying 1.4 litre of culture broth using the procedure described in Example 1b, 18 ml of a solution of the partially purified enzyme having the activity 540 GALU/ml and 32 EMANU/ml (2900 HCU/ml) was obtained.

### Example 3

Using the same procedure as described in Example 1a, with the proviso that the strain used was Penicillium notatum DSM 3216, the yield was 4 GALU/ml.

Purifying 1.1 litre of culture broth using the procedure described in Example 1b, 18 ml of a solution of the partially purified enzyme having the activity 300 GALU/ml and 8 EMANU/ml (700 HCU/ml) was obtained.

### Example 4

### a) Enzyme Production

This example illustrates the production of α-galactomannangalactosidase by Penicillium chrysogenum in a pilot plant fermentor. The seed culture consisted of 1 litre of the following medium: 50 g of soya molasses (Aarhus Oliefabrik, Denmark), 20 g of ammonium sulphate, 10 g of potassium dihydrogen phosphate, 1 g of magnesium sulphate, 5 g of calcium carbonate and 0.5 g of Pluronic. The pH was adjusted to 6.5 and the medium was sterilized in 3 litre Erlenmeyer flasks at 125°C for 40 minutes. After cooling to 30°C the medium was inoculated with spores of Penicillium chrysogenum from YPG agar slant which had been inoculated at 30°C for 7 days. The flasks were shaken at 30°C for 2 days. 800 ml of this seed culture was transferred to the main fermentor containing 400 g of soya molasses, 160 g of ammonium sulphate, 80 g of potassium dihydrogen phosphate, 8 g of magnesium sulphate, heptahydrate, 40 g of calcium carbonate, 3 g of Pluronic and water to a volume of 8 litre. The pH was adjusted to 6.5 and the medium was sterilized for 120 minutes at 125°C. 100 ml of 15% phosphoric acid was sterilized separately at 125°C for 30 minutes and was used to regulate the pH during the fermentation. Following the inoculation, aeration (8 litre/minute) was started. Stirring at a velocity of 520 - 830 rpm was also started. The temperature was kept constant at 30°C. The fermentation was maintained for 65 hours during which time the pH rose from 6.7 - 7.02. Thereafter, the tank was cooled and the mycelium was separated by centrifugation. The supernatant liquid was analysed and the activity found to be 3 GALU/ml.

### b) Enzyme Purification

11 litres of culture broth was filtered using 140 g of kieselguhr on a filtering cloth and the liquid was sucked through using a porcelain funnel with a diameter of 40 cm.

Concentration by osmosis was performed on a Millipore^{TM} Pelican cassette with a cut off on molecular weight 10,000. The filter type used was polysulfone with the code PTGC 00005. Thereby, 11 litre were concentrated to 1 litre. Washing out was made with deionized water until the conductance was below 3 mSi.

The solution concentrated by osmosis was frozen and thawed whereafter the pH was adjusted to 7.0 The solution was filtered on a glass filter (Whatman^{TM} GF/D).

Thereafter, the filtrate was subjected to ion exchange chromatography on a DEAE-Tris acryl column followed by a concentration using an Amicon Hollow fibre cartridge (PDP 10) analagously as described in Example 1b. Thereby, 160 ml of a solution of a partially purified enzyme having the activity 20 GALU/ml and below 3 EMANU/ml (below 250 HCU/ml) was obtained.

### Example 5

This example illustrates the production of α-galactomannangalactosidase by Penicillium islandicum in a pilot plant fermentor.

The strain was cultivated at 30°C for 1 week on a YPG agar slant. The spores from the slant were used to inoculate the seed fermentor which consisted of 1 litre medium of the following composition: 20 g of cornsteep liquor, 10 g of ammonium nitrate, 10 g of potassium dihydrogen phosphate, 5 g of calcium carbonate, 20 g of raffinose, 1 g of magnesium sulphate, heptahydrate, 0.1 g of Pluronic and tap water to a volume of 1 litre. The pH was adjusted to 6.5. The medium was sterilized at 125°C for 60 minutes. The seed culture was shaken at 30°C for 2 days at 230 rpm. 800 ml of this seed culture was used to inoculate the main fermentor which consisted of 8 litre of the following substrate: 160 g of cornsteep liquor, 80 g of ammonium nitrate, 80 g of potassium dihydrogen phosphate, 8 g of magnesium sulphate, heptahydrate, 160 g of raffinose, 40 g of calcium carbonate, 1 g of Pluronic and tap water to a volume of 8 litre. The pH was adjusted to 6.5. The medium was sterilized at 125°C for 120 minutes. When the temperature fell to 30°C, it was inoculated with 800 ml of seed culture. The temperature was maintained at 30°C, agitation at 620 rpm, pressure at 0.5 ato, aeration at 8 litre/minute and the pH fell from 6.45 to 5.9. After 90 hours, the fermentation was stopped, cooled and centrifuged. The supernatant contained 7 GALU/ml.

Purifying 8 litre of culture broth using the procedure described in Example 4b, 500 ml of a solution of the partially purified enzyme having the activity 60 GALU/ml and 1.4 EMANU/ml (130 HCU/ml) was obtained.

### Example 6

In this example the α-galactomannangalactosidase was produced by Penicillium notatum. The strain was grown on a YPG agar slant at 30°C for 1 week. The spores were used to inoculate 1 litre of substrate of the following composition: 20 g of cornsteep liquor, 10 g of ammonium nitrate, 10 g of potassium dihydrogen phosphate, 1 g of magnesium sulphate, heptahydrate, 5 g of guar gum, 20 g of raffinose, 5 g of calcium carbonate, 0.1 g of Pluronic and tap water to a volume of 1 litre. The pH was adjusted to 6.5 and the medium was sterilized at 125°C for 40 minutes. The flask was shaken at 230 rpm for 2 days at 30°C after which time 800 ml was used to inoculate the main fermentor containing the following substrate: 160 g of cornsteep liquor, 80 g of ammonium nitrate, 80 g of potassium dihydrogen phosphate. 8 g of magnesium sulphate, heptahydrate, 40 g of guar gum, 40 g of calcium carbonate, 160 g of raffinose, 1 g of Pluronic and tap water to a volume of 8 litre. The pH was adjusted at 6.5. Growth was maintained for 89 hours during which time the pH rose from 6.25 to 6.95. The aeration was 8 litre/minute, the pressure 0.5 ato and the agitation 520 - 830 rpm. At the end of the fermentation the tank was cooled and the broth centrifuged to remove the mycelium. The supernatant contained 13 GALU/ml.

Purifying 7,5 litre of culture broth using the procedure described in Example 4b, 40 ml of a solution of the partially purified enzyme having the activity 660 GALU/ml and 93 EMANU/ml (8400 HCU/ml) was obtained.

### Example 7

This example illustrates the preparation of a standard substrate hereinafter designated guar G. This substrate is well suited for discrimination of α-galactomannangalactosidase from α-galactosidases which lack the ability to attack galactomannans with intact mannan backbone.

1 litre of tap water was heated to 75°C and 10 g of guar flour was added with stirring. 16 ml of Gamanase 1.5 L was added and the pH was adjusted to 5.0. 190 g of guar flour was added during one hour with stirring. Areas of the galactomannan not protected by D-galactopyranosyl groups were, under these conditions, cleaved by the endo-β-mannanase present in the Gamanase and any α-galactomannangalactosidase present was heat inactivated at 75°C. The hydrolysis was stopped by boiling. The modified guar G was purified by precipitation with 500 ml 96% (weight/volume) alcohol, filtered and dried. The yield was 140 g of guar G.

### Example 8

This example illustrates the initiation rate, i.e., the release of galactose from the standard screening substrate, guar G (see Example 7).

All the partially purified enzymes tested were diluted in 50 mM sodium acetate buffer (pH: 5.5) and, thereafter incubated with guar G in a concentration of 4% (weight/volume) for 30 and 60 minutes at 45°C. The release of galactose was determined using the enzymatic galactose UV-method from Boehringer Mannheim with their kit.

The results obtained appear in table I, below.

**Table I**

| Enzyme from example No. | Concentration tested, GALU/ml | Initiation rate, galactose liberated, g/(litre x hour x GALU) |
|---|---|---|
| 1 + 4 | 0.02 | 1.4 |
| 1 + 4 | 0.05 | 1.3 |
| 2 + 5 | 0.02 | 0.4 |
| 2 + 5 | 0.05 | 0.3 |
| 3 + 6 | 0.02 | 1.2 |
| 3 + 6 | 0.05 | 1.0 |

Testing coffee bean α-galactosidase (Sigma No. G 8507) in a concentration of 0.05 GALU/ml, the initiation rate was 0.5 g galactose/(litre x hour x GALU).

Using Aspergillus niger-α-galactosidase obtained from Sigma (No. G 9007) in a concentration of 0.05 GALU/ml, the initiation rate was less than 0.5 g galactose/(litre x hour x GALU) in this test.

Also the following fungal α-galactosidases were unable to release galactose from guar G in this test: Aspergillus aculeateus, Aspergillus oryzae, Cephalosporium acremonium, Fomes unguleatus, Irpex lacteus, Penicillium aculeatum, Penicillium adametzi, Penicillium crustosum, Penicillium mellinii, Penicillium oxalicum, Penicillium simplicissimum, Pycnoporus cinnabarius, Pycnoporus coccinus, Pycnoporus sanguineus, Streptomyces libani, Streptomyces sioyaensis, Trametes hirsuita and Tricoderma harzianum.

### Example 9

This example illustrates the release of galactose from guar flour. The enzyme used was the Penicillium chrysogenum α-galactomannangalactosidase prepared as described in Example 4. The amount of galactose released was determined as described in Example 8 using the Boehringer Mannheim kit and the amount of galactose released is expressed as mg galactose released per g galactose flour. The incubation was performed in a water bath with temperature control and air tight lid. The guar flour tested was wetted with 20 GALU of the enzyme per g guar flour and with an acetate buffer giving a final concentration of 40% (weight/weight) guar flour. The results obtained appears from Table II.

**Table II**

| Temperature, °C | pH | Release of galactose, mg/g guar flour | |
|---|---|---|---|
| | | 5 hours incubation | 24 hours incubation |
| 45 | 4.5 | 80 | 140 |
| 55 | 4.5 | 80 | 80 |
| 45 | 5.0 | 80 | 140 |
| 55 | 5.0 | 80 | 110 |
| 45 | 5.5 | 90 | 160 |
| 55 | 5.5 | 90 | 110 |

As appears from table II, the enzyme is not stable for long time at temperatures above 50°C. The pH optimum of the enzyme is between 5.0 and 5.5

The effect of the concentration of the enzyme was tested by incubation analogously as described above using the temperature 45°C, a pH of 5.0 and the enzyme concentrations stated in Table III which also shows the results obtained.

**Table III**

| Enzyme dosis GALU/g guar flour | Release of galactose, mg/g guar flour | |
|---|---|---|
| | 5 hours incubation | 24 hours incubation |
| 2 | 25 | 65 |
| 4 | 38 | 80 |
| 16 | 75 | 130 |
| 50 | 110 | 190 |
| 90 | 116 | 220 |

### Example 10

This example illustrates affinity purification of the enzymes obtained in Examples 2 and 4 through 6.

30 ml of partly purified α-galactomannangalactosidase from Example 4 was purified to be free from endo-β-mannanase activity by the following procedure: 100 ml galactosamin (Sigma No. G 0500) coupled to AH-Seharose^{TM} (Pharmacia, Sweden) was equilibrated with a buffer (50 mM sodium acetate (pH: 5.0)). The 30 ml solution was applied and the endo-β-mannanase and some of the α-galactomannangalactosidase ran through the column. The α-galactomannangalactosidase was eluted with a sodium chloride gradient. The α-galactomannangalactosidase purified free of endo-β-mannanase had a specific activity of 15 GALU per mg protein and endo-β-mannanase was below the detection limit. The molecular weight of the main protein band after SDS gel electrophoresis was found to be between 55,000 and 60,000. (SDS is sodium dodecyl sulphate.) The pH optimum was between about 5 and 6, the temperature optimum was about 50°C with p-NPGal as substrate, and the pI (isoelectric point) was 4.3.

The other enzymes from Examples 5 and 6 were purified using the same procedure. It was found that the α-galactomannangalactosidase from Example 6 had a molecular weight between 55,000 and 60,000, a specific activity of 75 GALU per mg protein, the pH optimum was between about 5 and 6, the temperature optimum was about 50°C with p-NPGal as substrate and the pI was about 4.3.

The α-galactomannangalactosidase from Examples 2 and 5 had a molecular weight between 62,000 and 67,000 and a specific activity of 90 GALU per mg protein. The pH optimum of this enzyme was between about 4 and 5.5. The pI was about 4.3.

For all three enzymes using guar substrate, the temperature optimum for 2 hours' incubation was 55°C and for 24 hours' incubation it was 50°C.

An amount of the highly purified enzyme from Example 4 corresponding to 4 GALU was dissolved in 3 ml of 50 mM sodium acetate buffer (pH: 5.0) and 2 g of guar flour was added. The resulting mixture was incubated for 20 hours at 45°C. Thereafter, the product was dried. It was found that 140 mg of galactose had been split off during the incubation with this enzyme.

Using the gelling test described In Example 11, below, it was observed that all the α-galactomannangalactosidase products prepared could be used to prepare galactomannans having improved gelling capacity.

### Example 11

This example illustrates a test for gelling capability.
a) 40 mg of xanthan (Sigma No. G 1253) was suspended in 10 ml of deionized water and then boiled to dissolve the material. Analagously, in a second glass, 40 mg of locust bean gum (Sigma No. G 0753) was suspended in 10 ml of deionized water and boiled to dissolve the material. At 75°C, 2 ml of each of the solutions were mixed in a 10 ml test tube, whereafter the tube was allowed to cool in an ice bath. 15 minutes later, the tube was turned upside down and the mixture did not run out.
b) Using guar gum (Sigma No. G 4129) instead of locust bean gum in the test described in a), above, the mixture ran out when the tube was turned upside down.
c) Using guar gum which had been treated with Aspergillus niger-α-galactosidase, instead of locust bean gum in the test described in a), above, the mixture ran out when the tube was turned upside down.
d) Using guar gum which had been treated with an α-galactomannangalactosidase according to this invention, instead of locust bean gum in the test described in a), above, the gel did not run out when the tube was turned upside down.

The deposits DSM 3214, DSM 3215 and DSM 3216 were made at the Deutsche Sammlung von Mikroorganismen on 22nd January 1985 and are governed by the Budapest Treaty. These strains were used in foregoing Examples 4, 5 and 6, respectively.

The features disclosed in the foregoing description and in the following claims may, both separately and in any combination thereof, be material for realising the invention in diverse forms thereof.

## Claims

1. A microbial α-galactomannangalactosidase having a molecular weight between 55,000 and 67,000, a pH optimum in the range of from 4 to 6, a pI value of about 4.3, a temperature optimum of 55°C (short term) and 50°C (long term) and having been produced by a strain selected from Penicillium chrysogenum, Penicillium islandicum and Penicillium notatum or mutants thereof capable of producing α-galactomannangalactosidase.

2. An α-galactomannangalactosidase product having at least about 250 GALU per gram of liquid or solid product form and being essentially free from endo-β-mannanase activity, the α-galactomannangalactosidase having a molecular weight between 55,000 and 67,000, a pH optimum in the range of from 4 to 6, a pI value of about 4.3, a temperature optimum of 55°C (short term) and 50°C (long term) and having been produced by a strain selected from Penicillium chrysogenum, Penicillium islandicum and Penicillium notatum or mutants thereof capable of producing α-galactomannangalactosidase.

3. The α-galactomannangalactosidase product of Claim 2, wherein the endo-β-mannanase activity is less than about 0.001 EMANU per GALU.

4. The microbial α-galactomannangalactosidase having a molecular weight between 55,000 and 67,000, a pH optimum in the range of from 4 to 6, a pI value of about 4.3, a temperature optimum of 55°C (short term) and 50°C (long term) elaborated extracellularly by cultivation of a microorganism strain productive of said enzyme selected from Penicillium chrysogenum, Penicillium islandicum and Penicillium notatum or mutants thereof capable of producing α-galactomannangalactosidase and thereafter recovered from the culture broth.

5. The method for producing α-galactomannangalactosidase having a molecular weight between 55,000 and 67,000, a pH optimum in the range of from 4 to 6, a pI value of about 4.3, a temperature optimum of 55°C (short term) and 50°C (long term) which comprises cultivating by submerged cultivation an α-galactomannangalactosidase producing strain selected from Penicillium chrysogenum, Penicillium islandicum and Penicillium notatum or mutants thereof capable of producing α-galactomannangalactosidase and thereafter recovering the enzyme from the culture medium.

6. The use of a product according to Claim 2 for modifying the galactomannan of guar gum, locust bean gum and the like by treating the gum in solution with the product having less than about 0.01 EMANU of endo-β-mannanase per GALU until at least about 10% of the galactose content in the gum has split off and thereafter halting the treatment.

## Patentansprüche

1. Mikrobielle α-Galactomannangalactosidase mit einem Molekulargewicht zwischen 55.000 und 67.000, einem pH-Optimum im Bereich von 4 bis 6, einem pI-Wert von etwa 4,3, einem Temperatur-Optimum von 55°C (Kurzzeit) und 50°C (Langzeit) und hergestellt durch einen Stamm, der ausgewählt ist aus Penicillium chrysogenum, Penicillium islandicum und Penicillium notatum oder Mutanten derselben, die in der Lage sind, α-Galactomannangalactosidase zu produzieren.

2. α-Galactomannangalactosidase-Produkt mit wenigstens etwa 250 GALU pro g flüssiger oder fester Produktform und im wesentlichen frei von endo-β-Mannanase-Aktivität, wobei die α-Galactomannangalactosidase ein Molekulargewicht zwischen 55.000 und 67.000, ein pH-Optimum im Bereich von 4 bis 6, einen pI-Wert von etwa 4,3, ein Temperatur-Optimum von 55°C (Kurzzeit) und 50°C (Langzeit) besitzt und hergestellt worden ist durch einen Stamm, der ausgewählt ist aus Penicillium chrysogenum, Penicillium islandicum und Penicillium notatum oder Mutanten derselben, die in der Lage sind, α-Galactomannangalactosidase zu produzieren.

3. α-Galactomannangalactosidase-Produkt nach Anspruch 2, dadurch gekennzeichnet, daß die endo-β-Mannanase-Aktivität weniger als etwa 0,001 EMANU pro GALU beträgt.

4. Mikrobielle α-Galactomannangalactosidase mit einem Molekulargewicht zwischen 55.000 und 67.000, einem pH-Optimum im Bereich von 4 bis 6, einem pI-Wert von etwa 4,3, einem Temperaturoptimum von 55°C (Kurzzeit) und 50°C (Langzeit), die extrazellulär durch Kultivierung eines Mikroorganismenstammes dargestellt, der besagtes Enzym produziert und ausgewählt ist aus Penicillium chrysogenum, Penicillium islandicum und Penicillium notatum oder Mutanten derselben, die in der Lage sind, α-Galactomannangalactosidase zu produzieren, und anschließend aus der Kulturbrühe gewonnen ist.

5. Vorfahren zur Herstellung von α-Galactomannangalactosidase mit einem Molekulargewicht zwischen 55.000 und 67.000, einem pH-Optimum im Bereich von 4 bis 6, einem pI-Wert von etwa 4,3, einem Temperatur-Optimum von 55°C (Kurzzeit) und 50°C (Langzeit), welches das Kultivieren durch Submers-Kultivierung eines α-Galactomannangalactosidase produzierenden Stamms, ausgewählt aus Penicillium chrysogenum, Penicillium islandicum und Penicillium notatum oder Mutanten derselben, die in der Lage sind, α-Galactomannangalactosidase zu produzieren, und anschließend die Gewinnung des Enzyms aus dem Kulturmedium umfaßt.

6. Verwendung eines Produkts nach Anspruch 2 zur Modifizierung des Galactomannans von Guar-Gummi, Johannesbrotgummi und dergleichen durch Behandeln des Gummis in Lösung mit dem Produkt mit weniger als etwa 0,01 EMANU endo-β-Mannanase pro GALU, bis zumindest etwa 10 % des Galactose-Gehalts im Gummi abgespalten worden sind, und anschließendes Stoppen der Behandlung.

## Revendications

1. α-galactomannangalactosidase microbienne ayant un poids moléculaire entre 55 000 et 67 000, un pH optimum dans la plage allant de 4 à 6, une valeur pI d'environ 4,3, un optimum de température de 55°C (court terme) et de 50°C (long terme) et ayant été produite par une souche choisie à partir de Penicillium chrysogenum, Penicillium islandicum et Penicillium notatum ou leurs mutants capables de produire l'α-galactomannangalactosidase.

2. Produit d'α-galactomannangalactosidase ayant au moins environ 250 unités GAL par gramme de produit sous forme liquide ou solide et étant sensiblement exempt d'activité endo-β-mannanase, l'α-galactomannangalactosidase ayant une poids moléculaire entre 55 000 et 67 000, un optimum de pH dans la plage allant de 4 à 6, une valeur pI d'environ 4,3, un optimum de température de 55°C (court terme) et 50°C (long terme) ayant été produite par une souche choisie à partir de Penicillium chrysogenum, Penicillium islandicum et Penicillium notatum ou leurs mutants capables de produire l'α-galactomannangalactosidase.

3. Produit d'α-galactomannangalactosidase selon la revendication 2, dans lequel l'activité d'endo-β-mannanase est inférieure à environ 0,001 unité EMAN par unité GAL.

4. α-galactomannangalactosidase microbienne ayant un poids moléculaire entre 55 000 et 67 000, un optimum de pH dans la plage allant de 4 à 6, une valeur pI d'environ 4,3, un optimum de température de 55°C (court terme) et de 50°C (long terme) élaborée par voie extracellulaire par mise en culture d'une souche de microorganismes, souche productive de l'enzyme choisie à partir de Penicillium chrysogenum, Penicillium islandicum et Penicillium notatum ou leurs mutants capables de produire l'α-galactomannangalactosidase et récupérée ensuite du bouillon de culture.

5. Procédé pour la production d'α-galactomannangalactosidase ayant un poids moléculaire entre 55 000 et 67 000, un optimum de pH dans la plage allant de 4 à 6, une valeur pI d'environ 4,3, un optimum de température de 55°C (court terme) et de 50°C (long terme) qui comprend la mise en culture par culture immergée d'une souche produisant l'α-galactomannangalactosidase choisie parmi Penicillium chrysogenum, Penicillium islandicum et Penicillium notatum ou leurs mutants capables de produire l'α-galactomannangalactosidase et à récupérer ensuite l'enzyme à partir du milieu de culture.

6. Utilisation d'un produit selon la revendication 2 destiné à modifier le galactomannan de la gomme guar, la gomme de caroube et analogue en traitant la gomme en solution avec le produit ayant moins d'environ 0,01 unités EMAN d'endo-β-mannanase par unité GAL jusqu'à ce qu'au moins environ 10 % de la teneur en galactose dans la gomme se soit séparé et après quoi on interrompt le traitement.
